(19) Europäisches Patentamt
European Patent Office
Office européen des brevets

(11) Numéro de publication: **0 031 282 B1**

(12) **FASCICULE DE BREVET EUROPEEN**

(45) Date de publication du fascicule du brevet: **16.05.84**

(51) Int. Cl.³: **C 07 D 213/89**

(21) Numéro de dépôt: **80401805.9**

(22) Date de dépôt: **16.12.80**

(54) **Cristaux moléculaires constitués de dérivés substitués à radical chiral de la nitropyridine-N-oxyde.**

(30) Priorité: **21.12.79 FR 7931454**

(43) Date de publication de la demande: **01.07.81 Bulletin 81/26**

(45) Mention de la délivrance du brevet: **16.05.84 Bulletin 84/20**

(84) Etats contractants désignés: **DE GB NL**

(56) Documents cités: **neant**

**Le dossier contient des informations techniques présentées postérieurement au dépôt de la demande et ne figurant pas dans le présent fascicule.**

(73) Titulaire: **Chemla, Daniel, 50, Avenue Jean Jaurès, F-92290 Châtenay-Malabry (FR)**
Titulaire: **Oudar, Jean-Louis, 27, Avenue du Plessis, F-92290 Châtenay-Malabry (FR)**
Titulaire: **Tsoucaris, Georges, 13, rue André-Theuriet, F-92340 Bourg-la-Reine (FR)**

(72) Inventeur: **Chemla, Daniel, 50, Avenue Jean Jaurès, F-92290 Châtenay-Malabry (FR)**
Inventeur: **Oudar, Jean-Louis, 27, Avenue du Plessis, F-92290 Châtenay-Malabry (FR)**
Inventeur: **Tsoucaris, Georges, 13, rue André-Theuriet, F-92340 Bourg-la-Reine (FR)**

(74) Mandataire: **Mongrédien, André et al, c/o BREVATOME 25, rue de Ponthieu, F-75008 Paris (FR)**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

EP 0 031 282 B1

BUNDESDRUCKEREI BERLIN

Cristaux moléculaires constitués de derivés substitués à radical chiral de la nitropyridine-N-oxyde

La présente invention a pour objet des cristaux moléculaires constitués de dérivés substitués à radical chiral de la nitropyridine N oxyde. Elle trouve une application en optique non linéaire et notamment en conversion de fréquences optiques, en électrooptique (notamment en modulation d'un rayonnement par un champ électrique), ainsi que dans l'obtention d'effets Kerr statique et optique et dans tous les dispositifs optoélectroniques où ces effets peuvent être exploités.

On connaît déjà de nombreux matériaux utilisables en optique non linéaire et en électrooptique. On peut citer, par exemple, pour les cristaux doubleurs de fréquences, le KDP (diphosphate de potassium) et, pour les cristaux électrooptiques, le niobate de lithium.

Ces matériaux souffrent d'un inconvénient qui est leur manque d'efficacité, ce qui oblige à les employer sous de fortes épaisseurs. L'invention a justement pour objet un matériau nouveau dont l'efficacité est améliorée, ce qui permet d'obtenir des effets semblables avec des épaisseurs moindres ou des effets supérieurs à épaisseurs égales.

A cette fin, l'invention propose des produits nouveaux, utilisés à l'état de cristaux et qui sont des dérivés substitués particuliers de la nitropyridine N oxyde. La 4-nitropyridine N-oxyde a pour formule:

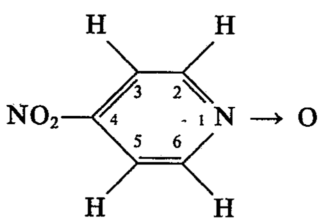

Les dérivés de l'invention sont caractérisés en ce qu'au moins un radical chiral R (c'est-à-dire non superposable à son image prise dans un miroir plan) est greffé en position ortho, méta ou para.

Naturellement, l'invention couvre également les dérivés analogues de la 5-nitropyridine N-oxyde bien que ce corps soit moins avantageux en général du fait de l'opposition moins marquée des groupements $NO_2$ et $N \rightarrow O$.

Selon l'invention le radical chiral R est un carbone relié à trois radicaux différents $R_1$, $R_2$, $R_3$, les radicaux $R_1$, $R_2$, $R_3$ représentant un atome d'hydrogène ou un radical pouvant être pris dans le groupe alkyle ou un radical $COOR_4$ où $R_4$ représente un atome d'hydrogène ou un radical alkyle.

Les demandeurs pensent pouvoir expliquer l'efficacité remarquable de ces produits nouveaux de la manière suivante. Les cristaux destinés à l'optique non linéaire et aux autres applications mentionnées plus haut doivent satisfaire à deux conditions:

1. être formés d'unités microscopiques présentant de fortes non linéarités optiques,
2. avoir des structures cristallines telles que les contributions microscopiques s'ajoutent constructivement; il faut donc que la structure moléculaire soit non centrée et que les unités microscopiques soient relativement bien alignées.

Dans l'art antérieur, la condition 1 est remplie par l'emploi de corps dont les unités microscopiques présentent des dipôles électriques permanents élevés. Cependant, l'existence même de ces dipôles fait apparaître des forces qui favorisent les structures cristallines dans lesquelles les dipôles permanents s'opposent, ce qui nuit à l'alignement des unités microscopiques. D'où des compensations qui affaiblissent les non linéarités macroscopiques du cristal. Les conditions 1 et 2 sont donc rarement satisfaites simultanément avec les produits de l'art antérieur.

Les inventeurs ont découvert que les dérivés substitués mentionnés de la nitropyridine-N-oxyde présentent une non linéarité très élevée et simultanément un dipôle permanent très faible. Pour ces molécules, en effet, le dipôle créé par les électrons $\pi$ (électrons qui gouvernent la non linéarité de la molécule) est opposé au dipôle créé par les électrons $\sigma$, avec une amplitude analogue, de sorte que le dipôle total de la molécule est très faible alors que la non linéarité est élevée. Le phénomène de compensation des matériaux de l'art antérieur disparaît donc et la non linéarité peut être conservée à l'échelle microscopique. De plus, la présence du radical chiral permet de remplir au mieux la condition 2.

En ce qui concerne les procédés d'obtention et de caractérisation de ces corps, ils peuvent être analogues à ceux relatifs de la nitropyridine-N-oxyde ou à ses dérivés substitués symétriques (comme la méthyl ou diméthyl-nitropyridine-N-oxyde). Ces procédés sont décrits notamment dans:

- l'article de MASUMI YAMAKAWA et al intitulé »Absorption and phosphorescence spectra of 4-nitropyridine-N-oxides and 4-and 3-nitroquinoline-N-oxides« publié dans la revue »Spectrochimica Acta«, vol. 30, p. 2103—2119, 1974, et dans
- l'article de MOTOO SHIRO et al. intitulé »The crystal and Molecular Structures of 3-methyl-4-nitropyridine-N-oxide and 3-5-dimethyl-4-nitropyridine-N-oxide« publié dans la revue »Acta crystallographica« B 33, 1977, 1549—1556.

## Revendication

Cristaux moléculaires, caractérisés en ce qu'ils sont constitués de molécules de dérivés de la 4 ou 5 nitropyridine-N-oxyde substitué par au

moins un radical chiral greffé en position ortho, méta ou para, ce radical étant constitué par un carbone asymétrique de formule:

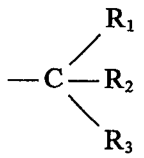

dans laquelle $R_1$, $R_2$, $R_3$ sont tous différents et représentent un atome d'hydrogène, ou un radical alkyle, ou $COOR_4$ où $R_4$ représente un atome d'hydrogène ou un radical alkyle.

## Patentanspruch

Molekülkristall, dadurch gekennzeichnet, daß er von Molekülen von Derivaten von 4- oder 5-Nitropiridin-N-Oxid besteht, das mit wenigstens einem chiralen Radikal substituiert ist, das in der Ortho-, Meta- oder Para-Stellung angeordnet ist und von einem asymmetrischen Kohlenstoff der Formel

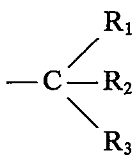

gebildet ist, wobei $R_1$, $R_2$ und $R_3$ alle verschieden und ein Wasserstoffatom oder ein Alkylrest oder $COOR_4$ sind, mit $R_4$ einem Wasserstoffatom oder einem Alkylrest.

## Claim

Molecular crystals characterized in that they comprise molecules of 4- or 5-nitropyridine-N-oxide derivatives substituted in the ortho-, meta or para-position by at least one chiral radical, said radical comprising an asymmetric carbon atom of the formula

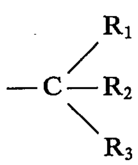

wherein $R_1$, $R_2$ and $R_3$ are all different, and each represents a hydrogen atom, an alkyl group, or $-COOR_4$, where $R_4$ represents a hydrogen atom or an alkyl group.